# DEMANDE DE BREVET EUROPEEN

(11) **EP 1 145 705 A2**
(43) Date de publication de la demande: **17.10.2001**
(21) Numéro de dépôt: 01400672.0
(22) Date de dépôt: 14.03.2001
(51) Int. Cl.: A61K 7/06, A61K 7/48

(54) **Composition, notamment cosmétique, renfermant un stéroide et un 2-alkyl alcanol ou un ester**

(30) Priorité: 10.04.2000 FR 0004576
(71) Demandeur: L'OREAL, 75008 Paris (FR)
(72) Inventeur: Baldo, Francine, 92330 Sceaux (FR); Dreher, Susanne, 75020 Paris (FR)
(74) Mandataire: Rasson, Catherine

(57) **Abrégé**

La présente invention concerne une composition renfermant au moins un stéroïde choisi parmi : la DHEA et/ou un précurseur biologique et/ou un dérivé chimique ou métabolique de celle-ci, caractérisée en ce qu'elle comprend en outre au moins un 2-alkyl alcanol comprenant de 12 à 36 atomes de carbone, ou un ester d'un tel alcool.

L'invention concerne également les utilisations cosmétiques et dermatologiques de cette composition, notamment pour prévenir ou traiter le vieillissement chronologique ou actinique et la canitie.

## Description

La présente invention se rapporte à une composition renfermant au moins un stéroïde choisi parmi: la DHEA et/ou un précurseur biologique et/ou un dérivé chimique ou métabolique de celle-ci et au moins un 2-alkyl alcanol comprenant de 12 à 36 atomes de carbone, ou un ester d'un tel alcool, à des utilisations de ladite composition et à un procédé de solubilisation du stéroïde précité au moyen d'au moins un 2-alkyl alcanol comprenant de 12 à 36 atomes de carbone, ou un ester d'un tel alcool.

La DHEA, ou déhydroépiandrostérone, est un stéroïde naturel produit essentiellement par les glandes corticosurrénales. La DHEA exogène, administrée par voie topique ou orale, est connue pour sa capacité à promouvoir la kératinisation de l'épiderme (JP-07 196 467) et à traiter les peaux sèches en augmentant la production endogène et la sécrétion de sébum et en renforçant ainsi l'effet barrière de la peau (US-4,496,556). La DHEA est également décrite dans le traitement des symptômes de la ménopause (US-5,854,671) et dans la prévention et le traitement de l'ostéoporose (US-5,776,923). Il a aussi été suggéré d'utiliser la DHEA dans le traitement de l'obésité et du diabète (WO 97/13500), des maladies cardiovasculaires (US-5,854,671) et dans le traitement de certains cancers, tels que le cancer de l'ovaire (US-5,798,347), de l'utérus (US-5,872,114) ou du sein (US-5,824,671).

Or, la DHEA ne se solubilise que difficilement dans les milieux aqueux et hydroalcooliques, ce qui limite sa formulation dans des compositions cosmétiques ou dermatologiques appliquées par voie topique ou orale. Elle a ainsi tendance à recristalliser ou à se dégrader. Il s'ensuit une perte d'efficacité plus ou moins importante de ces compositions, selon le degré de recristallisation et/ou de dégradation, ce qui va à l'encontre de l'objectif recherché. En outre, cette recristallisation ou dégradation peut modifier la stabilité globale de ces compositions ainsi que leur aspect, ce qui peut détourner l'utilisateur de celles-ci.

Il subsiste donc le besoin de solubiliser la DHEA, ainsi que ses précurseurs et/ou dérivés posant les mêmes problèmes de recristallisation, dans un solubilisant physiologiquement acceptable.

La Demanderesse a maintenant constaté, de façon étonnante, que ces composés pouvaient être facilement solubilisés dans les alcools de Guerbet et leurs esters.

Les alcools dits de Guerbet sont des 2-alkyl alcanols répondant à la formule générale (II) suivante : 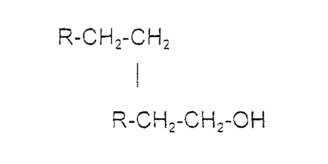 dans laquelle R désigne le même groupement aliphatique saturé.

Ces alcools sont généralement obtenus par condensation, à haute température et en présence de catalyseurs alcalins, de deux molécules d'aldéhyde pour former un aldol, puis par élimination d'eau. En raison de leur structure ramifiée, ces composés sont liquides à des températures extrêmement basses. En outre, du fait de leur poids moléculaire élevé, ils sont peu irritants, peu volatils et ont de bonnes propriétés lubrifiantes. Enfin, leur chaîne aliphatique saturée leur confère une très bonne stabilité vis-à-vis de l'oxydation à haute température.

Ces propriétés avantageuses expliquent que ces composés soient utilisés à ce jour dans de très nombreuses applications, et notamment comme émollients et agents de conditionnement dans des compositions cosmétiques très diverses.

Certains d'entre eux ont également été décrits comme solubilisants d'actifs tels que l'acide n-octanoyl-5-salicylique, l'acide caféique, l'acide kojique (EP 0 679 388) et le rétinol (EP 0 646 371 et EP 0 666 076).

Toutefois, à la connaissance de la Demanderesse, il n'a jamais été proposé d'utiliser les alcools de Guerbet pour solubiliser la DHEA, un précurseur biologique de celle-ci ou un dérivé chimique ou métabolique de celle-ci.

La présente invention a donc pour objet une composition renfermant au moins un stéroïde choisi parmi : la DHEA et/ou un précurseur biologique et/ou un dérivé chimique ou métabolique de celle-ci, caractérisée en ce qu'elle comprend en outre au moins un 2-alkyl alcanol comprenant de 12 à 36 atomes de carbone, ou un ester d'un tel alcool.

Elle a également pour objet un procédé de solubilisation d'au moins un stéroïde choisi parmi : la DHEA et/ou un précurseur biologique et/ou un dérivé chimique ou métabolique de celle-ci, comprenant l'étape consistant à mélanger ledit stéroïde à au moins un 2-alkyl alcanol comprenant de 12 à 36 atomes de carbone, ou à un ester d'un tel alcool. Le mélange peut être effectué à froid, à température ambiante ou à chaud, par exemple à 75°C, généralement sous agitation.

Selon un mode de réalisation préféré, ce procédé comprend en outre les étapes successives consistant à : (a) ajouter au mélange obtenu précédemment une composition comprenant des composés susceptibles de former des phases lamellaires, de manière à obtenir une phase huileuse ; (b) mélanger éventuellement cette phase huileuse à une phase aqueuse, pour réaliser une émulsion ; (c) soumettre la phase huileuse obtenue selon l'étape (a) ou l'émulsion obtenue selon l'étape (b) à une homogénéisation haute pression de manière à obtenir une composition comprenant des globules huileux enrobés de phases lamellaires ; et (d) ajouter éventuellement au moins un gélifiant à la composition obtenue selon l'étape (c).

La Demanderesse a en effet découvert que la formation de globules huileux enrobés de phases lamellaires, encore appelés "oléosomes", tels que décrits dans les demandes EP-0 641 557 et EP-0 705 593, permettaient de retarder la cristallisation des compositions selon l'invention. Les composés susceptibles de former des phases lamellaires, qui peuvent être utilisés pour mettre en oeuvre le procédé ci-dessus, sont donc notamment décrits dans les deux demandes de brevet précitées. Ils comprennent, d'une manière générale, les associations d'un tensioactif lipophile -tel qu'un ester de polyol- avec un tensioactif hydrophile -tel qu'un ester de polyol polyoxyalkyléné- et un acide gras et/ou un lipide amphiphile ionique. Les oléosomes ont généralement un diamètre moyen inférieur à 200 nm.

La DHEA a la formule (I) suivante :

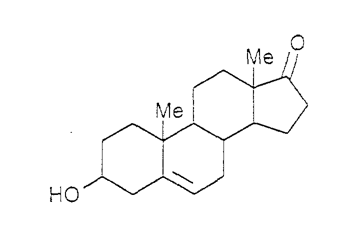

La DHEA utilisable selon l'invention est par exemple disponible auprès de la société AKZO NOBEL.

Par "précurseurs biologiques" de la DHEA, on entend notamment la Δ5-prégnénolone, la 17α-hydroxy prégnénolone et le sulfate de 17α-hydroxy prégnénolone, sans que cette liste soit limitative.

Par dérivés de la DHEA, on entend aussi bien ses dérivés métaboliques que ses dérivés chimiques. Comme dérivés métaboliques, on peut citer notamment le Δ5-androstène-3.17-diol et notamment le 5-androstène 3β, 17β-diol, la Δ4-androstène-3,17-dione, la 7 hydroxy DHEA (7α-hydroxy DHEA ou 7β-hydroxy-DHEA) et la 7-céto-DHEA qui est elle-même un métabolite de la 7β-hydroxy DHEA., sans que cette liste soit limitative.

La 7α-hydroxy DHEA est, avec le 5-androstène 3β, 17β-diol, un métabolite majeur de la DHEA, obtenu par action de la 7α-hydroxylase sur la DHEA. La 7β-hydroxy DHEA est un métabolite mineur de la DHEA, obtenu par action de la 7β-hydroxylase sur la DHEA. La 7-hydroxy DHEA pouvant être utilisée dans la composition de l'invention est de préférence la 7α-OH DHEA. Un procédé de préparation de ce composé est notamment décrit dans les demandes de brevet FR-2 771 105 et WO 94/08588. Toutefois, la 7β-OH DHEA convient également dans une composition selon la présente invention.

Comme dérivés chimiques, on peut citer notamment les sels de DHEA, en particulier les sels hydrosolubles, tels que le sulfate de DHEA. On peut citer également les esters, tels que les esters d'acides hydroxycarboxyliques et de DHEA décrits notamment dans US-5,736,537 ou les autres esters tels que le salicylate, l'acétate, le valérate (ou n-heptanoate) et l'énanthate de DHEA. Cette liste n'est évidemment pas limitative.

Les stéroïdes spécifiquement mentionnés ci-dessus sont compris dans le groupe des composés répondant à la formule (II): 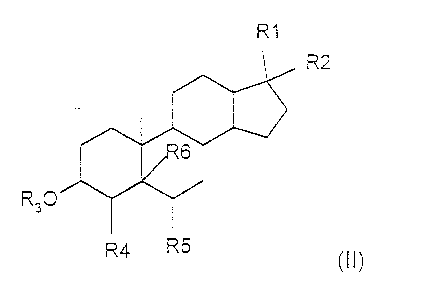 dans laquelle:
R₁ représente un atome d'hydrogène ou un groupe hydroxy,
R₂ est un radical -CO-CH₃ ou un groupe hydroxy,
ou R₁, R₂ forment ensemble un groupe oxo,
R₃ est un atome d'hydrogène ou un radical -COR' où R' représente un radical hydrocarboné linéaire, cyclique ou ramifié, saturé ou insaturé, éventuellement hydroxylé, en C₁-C₁₂, ou un radical aryle éventuellement hydroxylé, et
soit R₄ représente un atome d'hydrogène et R₅, R₆ forment ensemble une liaison,
soit R₄, R₅ forment ensemble une liaison et R₆ représente un atome d'hydrogène,
ainsi que leurs sels.

Par radical hydrocarboné, on entend par exemple les radicaux alcoyle tels que les radicaux méthyle, éthyle, propyle, iso-propyle, n-butyle, iso-butyle, sec-butyle, tert-butyle, pentyle, hexyle, heptyle, octyle, nonyle, décyle, undécyle et dodécyle, ainsi que les radicaux alcoylène, en particulier les radicaux vinyle ou allyle.

Par radical aryle, on entend de préférence le radical benzyle.

La composition selon la présente invention renferme, comme solubilisant des stéroïdes ci-dessus, au moins un 2-alkyl alcanol comprenant de 12 à 36 atomes de carbone, et de préférence de 16 à 20 atomes de carbone, ou un ester d'un tel alcool.

Comme 2-alkyl alcanols convenant à une mise en oeuvre dans la présente invention, on peut citer : le butyloctanol, le pentyl nonanol, l'hexyldécanol, l'octyldécanol, l'alcool isostéarylique, l'octyldodécanol, le undécylpentadécanol, le dodécylhexadécanol, le tétradécyloctadécanol, l'hexyldécyloctadécanol, le décyltétradécanol, le tétradécyleicosanol et le cétylarachidol.

Tous ces composés sont disponibles dans le commerce, auprès de CONDEA VISTA sous la dénomination commerciale lsofol® de EXXON CHEMICAL sous la dénomination Exxal® ou de JARCHEM sous la dénomination Jarcol® . On peut également utiliser le composé disponible auprès de HENKEL sous la dénomination commerciale EUTANOL G.

On peut également citer le mélange d'alcools iso-cétylique, iso-stéarylique et iso-arachidylique disponible auprès de CONDEA sous la dénomination commerciale Isofol 18T® .

En particulier, on préfère utiliser le 2-octyl dodécanol.

Comme esters desdits alcools, on peut citer un ester de 2-hexyl décanol et d'un acide choisi parmi : l'acide palmitique, l'acide caprylique, l'acide caprique, l'acide laurique, l'acide myristique et l'acide isostéarique ; un ester de 2-butyl octanol et d'un acide choisi parmi : l'acide thiodipropionique et l'acide trimellitique ; l'isostéarate de 2-décyl tétradécanol ; l'octanoate d'octyldodécyle ; et l'octyldodécyle meadowfoamate, qui est un ester d'octyldodécanol et d'acides gras dérivés d'huile de germe de *Limnanthes Alba*, disponible auprès de FANNING CORPORATION sous la dénomination commerciale Meadowester gme.

La concentration en stéroïdes dans la composition selon l'invention est avantageusement comprise entre 0,001% et 20% en poids, de préférence entre 0,01 et 10% en poids, plus préférentiellement entre 0,1 et 3% en poids, par rapport au poids total de la composition. En outre, la quantité pondérale de 2-alkyl alcanol ou de son ester est de préférence égale, plus préférentiellement au moins deux fois supérieure, à la quantité pondérale de stéroïdes. Elle représente avantageusement de 0,01% à 25%, mieux, de 1 à 15%, du poids total de la composition.

La composition selon l'invention peut se présenter sous toutes les formes galéniques normalement utilisées pour une application topique sur la peau, notamment sous forme d'une solution huileuse, d'une émulsion huile-dans-eau ou eau-dans-huile ou multiple, d'une émulsion siliconée, d'une microémulsion ou nanoémulsion, d'un gel huileux, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau, ou comme produit capillaire, par exemple comme shampooing ou après-shampooing.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules. Ces adjuvants, ainsi que leurs concentrations, doivent être tels qu'ils ne nuisent pas aux propriétés avantageuses des stéroïdes selon l'invention.

Lorsque la composition selon l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut utiliser, outre les solubilisants à base de 2-alkyl alcanols et de leurs esters, les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween® 20 ou Tween® 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Comme actifs, on peut utiliser notamment les dépigmentants et les agents kératolytiques et/ou desquamants.

En cas d'incompatibilité, les actifs indiqués ci-dessus et/ou les stéroïdes selon l'invention peuvent être incorporés dans des sphérules, notamment des vésicules ioniques ou non-ioniques et/ou des nanoparticules (nanocapsules et/ou nanosphères), de manière à les isoler les uns des autres dans la composition.

Selon une variante de l'invention, la composition peut être adaptée à une administration par voie orale. Dans ce cas, elle peut se présenter sous forme de sirops, de suspensions, de solutions ou d'émulsions.

La composition selon l'invention trouve en particulier une application dans la prévention et le traitement des signes du vieillissement chronologique ou actinique, ainsi que dans le traitement de certaines pathologies.

La présente invention concerne donc également l'utilisation cosmétique de la composition mentionnée ci-dessus pour prévenir ou traiter le vieillissement chronologique ou actinique, en particulier :
- pour prévenir ou réduire l'aspect papyracé de la peau, et/ou
- pour améliorer l'homogénéité de la couleur de la peau et/ou pour blanchir la peau et/ou raviver l'éclat du teint, et/ou
- pour traiter les rides et ridules, et/ou
- pour lutter contre le relâchement cutané, et/ou
- pour lutter contre ou prévenir l'atrophie de la peau, et/ou
- pour lutter contre la sécheresse de la peau.

Elle concerne aussi l'utilisation de cette composition pour le traitement cosmétique du cuir chevelu, en particulier pour prévenir ou traiter la canitie.

La présente invention concerne également l'utilisation de la composition mentionnée ci-dessus pour fabriquer une préparation destinée à atténuer les taches pigmentaires.

Elle concerne aussi l'utilisation de la composition mentionnée ci-dessus pour fabriquer une préparation destinée au traitement de la canitie.

Dans tous les cas, la composition selon l'invention et/ou la préparation obtenue à partir de celle-ci comprend une quantité efficace de stéroïde, suffisante pour obtenir l'effet recherché, et un milieu physiologiquement acceptable

L'invention sera maintenant illustrée par les exemples non limitatifs suivants. Dans ces exemples les quantités sont indiquées en pourcentage pondéral, sauf indication contraire.

### EXEMPLES

### Exemple 1 : mise en évidence de l'effet solubilisant des alcools de Guerbet

On prépare trois compositions C1 à C3 telles que définies dans le Tableau 1.

**TABLEAU 1**

| | C1 | C2 | C3 | C4 |
|---|---|---|---|---|
| Distéarate polyglycérolé (2 mol) | 2 % | 2 % | 2 % | 2 % |
| Mono-stéarate de PEG (8 OE) | 1,35 % | 1,35 % | 1,35 % | 1,35 % |
| Acide stéarique | 1 % | 1 % | 1 % | 1 % |
| Conservateurs | 1,35% | 1,35% | 1,35% | 1,35% |
| **2-octyldodécanol** | **0 %** | **5 %** | **10 %** | **20 %** |
| DHEA | 1 % | 1 % | 1 % | 1 % |
| Benzoate d'alcools en C₁₂₋₁₅ | 20 % | 15 % | 10 % | 0 % |
| Neutralisants | 0,45 % | 0,45 % | 0,45 % | 0,45 % |
| Propylène glycol | 10 % | 10 % | 10 % | 10 % |
| Gélifiant | 0,5 % | 0,5 % | 0,5 % | 0,5 % |
| Eau | qsp 100 % | qsp 100 % | qsp 100 % | qsp 100 % |

Les compositions C1 à C3 sont préparées de manière classique pour l'homme du métier. La composition C4 est préparée comme décrit dans l'Exemple 2 ci-après.

Après quinze jours à 4°C ou à température ambiante, la composition C1 qui est dépourvue de 2-octyldodécanol (alcool de Guerbet) comprend déjà des cristaux visibles au microscope sous lumière polarisée. En revanche, dans les compositions C2 à C4, qui comprennent respectivement 5, 10 et 20% en poids de 2-octyldodécanol, la DHEA est parfaitement solubilisée, ce qui se traduit par une absence de cristaux après deux mois à 4°C, à température ambiante, à 37°C ou à 45°C.

Il ressort donc clairement du Tableau 1 que le 2-octyldodécanol, ou un autre alcool ou ester d'alcool de Guerbet, permet de solubiliser la DHEA, ou un autre stéroïde.

Le Tableau 2 ci-dessous mentionne, à titre comparatif, la solubilité de la DHEA dans différents solubilisants classiques (le pourcentage de DHEA est calculé par rapport au poids total de DHEA et de solvant ; TA se réfère à la température ambiante) :

**TABLEAU 2**

| Solvant | %DHEA | Solubilité | Solubilité après un mois (TA) |
|---|---|---|---|
| Phénoxyéthanol | 20% | Soluble à TA | Cristaux |
| Propylène glycol | 15-20% | Insoluble à TA | Cristaux |
| | 5-10% | Soluble à 50°C | Cristaux |
| Dodécanol | 10% | Insoluble même à 50°C | Cristaux |
| Benzoate d'alcools en C₁₂₋₁₅ | 3% | Soluble à 50°C | Cristaux |

Il ressort du Tableau 2 ci-dessus que la DHEA n'est pas solubles dans certains solvants usuels comme le propylène glycol ou le dodécanol. Dans d'autres solvants, tels que le phénoxyéthanol ou le benzoate d'alcools en C₁₂-₁₅, elle n'est pas soluble pendant une durée suffisamment longue pour permettre la réalisation de compositions commercialement viables.

### Exemple 2 : composition cosmétique

| *Phase A1* | |
|---|---|
| 2-octyldodécanol | 20 % |
| DHEA | 1 % |

| *Phase A2* | |
|---|---|
| Distéarate polyglycérolé (2 mol) | 2 % |
| Mono-stéarate de PEG (8 OE) | 1,35 % |
| Acide stéarique | 1 % |
| Conservateur | 0,1 % |

| *Phase B* | |
|---|---|
| Conservateurs | 0.35 % |
| Neutralisants | 0,25% |
| Propylène glycol | 10 % |
| Eau | qsp 100 % |

| *Phase C* | |
|---|---|
| Gélifiant | 0,5 % |
| Neutralisant | 0,2 % |
| Eau | qsp |

Cette composition a été préparée de la manière suivante : les phases A1, A2 et B ont été préparées séparément par mélange de leurs constituants à chaud, sous agitation. Les phases A1 et A2 ont été mélangées à chaud, puis la phase B leur a été ajoutée. Le mélange ainsi obtenu a été transféré vers un homogénéisateur haute pression où il a été soumis à trois passages à 600 bars avant incorporation de la phase C.

Cette composition peut être utilisée en applications bi-quotidiennes pour prévenir ou traiter les signes du vieillissement tels que les rides, ridules, l'aspect papyracé de la peau, le relâchement cutané, la perte d'éclat du teint et le dessèchement de la peau.

## Revendications

1. Composition renfermant au moins un stéroïde choisi parmi : la DHEA et/ou un précurseur biologique et/ou un dérivé chimique ou métabolique de celle-ci, **caractérisée en ce qu'**elle comprend en outre au moins un 2-alkyl alcanol comprenant de 12 à 36 atomes de carbone, ou un ester d'un tel alcool.

2. Composition selon la revendication 1, **caractérisée en ce que** ledit stéroïde est choisi dans le groupe des composés répondant à la formule (II) : 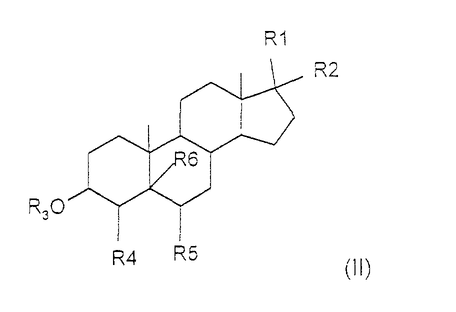 dans laquelle:
R₁ représente un atome d'hydrogène ou un groupe hydroxy,
R₂ est un radical -CO-CH₃ ou un groupe hydroxy,
ou R₁, R₂ forment ensemble un groupe oxo,
R₃ est un atome d'hydrogène ou un radical -COR' où R' représente un radical hydrocarboné linéaire, cyclique ou ramifié, saturé ou insaturé, éventuellement hydroxylé, en C₁-C₁₂, ou un radical aryle éventuellement hydroxylé, et
soit R₄ représente un atome d'hydrogène et R₅, R₆ forment ensemble une liaison, soit R₄, R₅ forment ensemble une liaison et R₆ représente un atome d'hydrogène,
ainsi que leurs sels.

3. Composition selon la revendication 2, **caractérisée en ce que** ledit radical hydrocarboné est choisi parmi les radicaux méthyle, éthyle, propyle, iso-propyle, n-butyle, iso-butyle, sec-butyle, tert-butyle, pentyle, hexyle, heptyle, octyle, nonyle. décyle, undécyle, dodécyle, vinyle et allyle.

4. Composition selon la revendication 2. **caractérisée en ce que** ledit radical aryle est le radical benzyle.

5. Composition selon l'une quelconque des revendications 1 à 4, **caractérisée en ce que** ledit précurseur biologique est choisi parmi : la Δ5-prégnénolone, la 17α-hydroxy prégnénolone et le sulfate de 17α-hydroxy prégnénolone.

6. Composition selon l'une quelconque des revendications précédentes, **caractérisée en ce que** ledit dérivé métabolique est choisi parmi : le Δ5-androstène-3,17-diol, la Δ4-androstène-3,17-dione, la 7 hydroxy DHEA et la 7-céto-DHEA.

7. Composition selon l'une quelconque des revendications précédentes, **caractérisée en ce que** ledit dérivé chimique est choisi parmi : les sels de DHEA et les esters de DHEA.

8. Composition selon la revendication 7, **caractérisée en ce que** ledit sel de DHEA est choisi parmi les sels hydrosolubles, tels que le sulfate de DHEA.

9. Composition selon la revendication 7, **caractérisée en ce que** ledit ester de DHEA est choisi parmi : les esters d'acides hydroxycarboxyliques et de DHEA ; le salicylate de DHEA ; l'acétate de DHEA ; le valérate de DHEA ; et l'énanthate de DHEA.

10. Composition selon l'une quelconque des revendications précédentes, **caractérisée en ce qu'**elle renferme de 0,01 à 10% en poids de stéroïde, par rapport au poids total de la composition.

11. Composition selon la revendication 10, **caractérisée en ce qu'**elle renferme de 0,1 à 3% en poids de stéroïde, par rapport au poids total de la composition.

12. Composition selon l'une quelconque des revendications précédentes, **caractérisée en ce que** ledit 2-alkyl alcanol comprend de 16 à 20 atomes de carbone.

13. Composition selon l'une quelconque des revendications précédentes, **caractérisée en ce que** ledit 2-alkyl alcanol est choisi parmi : le butyloctanol, le pentyl nonanol, l'hexyldécanol, l'octyldécanol, l'alcool isostéarylique, l'octyldodécanol, le undécylpentadécanol, le dodécylhexadécanol, le tétradécyloctadécanol, l'hexyldécyloctadécanol, le décyltétradécanol, le tétradécyleicosanol et le cétylarachidol et le mélange d'alcools iso-cétylique, iso-stéarylique et iso-arachidylique.

14. Composition selon la revendication 13, **caractérisée en ce que** ledit 2-alkyl alcanol est le 2-octyl dodécanol.

15. Composition selon l'une quelconque des revendications 1 à 11, **caractérisée en ce que** ledit ester de 2-alkyl acanol est choisi parmi : un ester de 2-hexyl décanol et d'un acide choisi parmi : l'acide palmitique, l'acide caprylique, l'acide caprique, l'acide laurique, l'acide myristique et l'acide isostéarique ; un ester de 2-butyl octanol et d'un acide choisi parmi : l'acide thiodipropionique et l'acide trimellitique ; l'isostéarate de 2-décyl tétradécanol ; l'octanoate d'octyldodécyle ; et l'octyldodécyle meadowfoamate.

16. Composition selon l'une quelconque des revendications précédentes, **caractérisée en ce qu'**elle comprend une quantité pondérale de 2-alkyl alcanol ou de son ester qui est au moins égale à la quantité pondérale de stéroïde.

17. Composition selon l'une quelconque des revendications précédentes, **caractérisée en ce qu'**elle comprend de 0,01% à 25% en poids de 2-alkyl alcanol ou de son ester.

18. Composition selon la revendication 17, **caractérisée en ce qu'**elle comprend de 1% à 10% en poids de 2-alkyl alcanol ou de son ester.

19. Utilisation cosmétique de la composition selon l'une quelconque des revendications précédentes pour prévenir ou traiter le vieillissement chronologique ou actinique.

20. Utilisation cosmétique de la composition selon l'une quelconque des revendications 1 à 18 pour prévenir ou réduire l'aspect papyracé de la peau et/ou pour améliorer l'homogénéité de la couleur de la peau et/ou pour blanchir la peau et/ou raviver l'éclat du teint, et/ou pour traiter les rides et ridules et/ou pour lutter contre le relâchement cutané, et/ou pour lutter contre ou prévenir l'atrophie de la peau, et/ou pour lutter contre la sécheresse de la peau.

21. Utilisation de la composition selon l'une quelconque des revendications 1 à 18 pour le traitement cosmétique du cuir chevelu, en particulier pour prévenir ou traiter la canitie.

22. Utilisation de la composition selon l'une quelconque des revendications 1 à 18 pour fabriquer une préparation destinée à atténuer les taches pigmentaires.

23. Utilisation de la composition selon l'une quelconque des revendications 1 à 18 pour fabriquer une préparation destinée au traitement de la canitie.

24. Procédé de solubilisation d'au moins un stéroïde choisi parmi : la DHEA et/ou un précurseur biologique et/ou un dérivé chimique ou métabolique de celle-ci, comprenant l'étape consistant à mélanger ledit stéroïde à au moins un 2-alkyl alcanol comprenant de 12 à 36 atomes de carbone, ou à un ester d'un tel alcool.

25. Procédé selon la revendication 24, **caractérisé en ce qu'**il comprend en outre les étapes successives consistant à : (a) ajouter au mélange du stéroïde et du 2-alkyl alcanol une composition comprenant des composés susceptibles de former des phases lamellaires, de manière à obtenir une phase huileuse ; (b) mélanger éventuellement cette phase huileuse à une phase aqueuse, pour réaliser une émulsion ; (c) soumettre la phase huileuse obtenue selon l'étape (a) ou l'émulsion obtenue selon l'étape (b) à une homogénéisation haute pression de manière à obtenir une composition comprenant des globules huileux enrobés de phases lamellaires ; et (d) ajouter éventuellement au moins un gélifiant à la composition obtenue selon l'étape (c).
